# EUROPEAN PATENT SPECIFICATION

(11) **EP 4 360 664 B1**
(45) Date of publication and mention of the grant of the patent: **09.12.2026**
(21) Application number: 23205197.9
(22) Date of filing: 23.10.2023
(51) Int. Cl.: A61L 9/12, A01M 1/00

(54) **ESSENCE CONTAINER WITH ROD STRUCTURE WITH A GRIPPING DEVICE**
ESSENZBEHÄLTER MIT STABSTRUKTUR MIT EINER GREIFVORRICHTUNG
CONTENEUR D'ESSENCE AVEC STRUCTURE DE TIGES ET DISPOSITIF DE PRÉHENSION

(30) Priority: 24.10.2022 ES 202231751 U
(43) Date of publication of application: 01.05.2024
(73) Proprietor: Vila Hermanos Cereria, S.A., 46869 Adzaneta De Albaida Valencia (ES)
(72) Inventor: GUY DOUENAT, Patrick, 46869 Adzaneta De Albaida (Valencia) (ES)
(74) Representative: Ungria López, Javier

(56) References cited:
- WO-A1-2007/098648
- CN-U- 207 714 885
- JP-U- 3 142 057
- KR-U- 20220 000 392

## Description

### Object of the invention

The present invention is directed to a set according to claim 1. The present disclosure relates to an essence diffusing rod structure with gripping device that arises from the need that exists when a diffuser container (bottle) has a large capacity (at least 500 ml), wherein the required diffusing sticks are too long compared to the diffuser container which must be of a larger format. Based on this premise, the novel gripping device of the disclosure makes it possible to have diffusing sticks of a shorter or equal length than the diffuser container, and thus be able to have a more compact diffuser container, which contributes to lower transport costs and consequently a clear environmental advantage.

### Background of the invention

Nowadays, essence diffusing sticks housed in diffuser containers and protruding above the mouthpieces thereof are known, for instance from KR 2022 0000392 U, so that when the diffusing sticks used are too long, the height of the containers is also greater, meaning that the space occupied by the containers and the sticks is greater, which results in a higher cost, with a higher transport cost due to the fact that more space is needed to house the different diffuser containers and the different sticks.

### Description

To achieve the objectives and avoid the drawbacks mentioned in the previous sections, the disclosure proposes an essence diffusing rod structure with a gripping device, said structure comprising two sticks that are joined together by the gripping device; wherein adjacent end sections of the two sticks are coupled to the gripping device. In the embodiment shown in the figures, the two sticks are arranged in one same longitudinal alignment.

The gripping device comprises a tubular body of revolution that includes a cylindrical inner recess; wherein the adjacent end sections of the two sticks of the rod structure fit tightly within said cylindrical inner recess.

In one embodiment of the disclosure the tubular body of revolution includes an outer surface with a convex curved generatrix.

The adjacent end sections of the two sticks of the rod structure include end portions that are in contact with each other.

In one embodiment of the disclosure the two sticks of the rod structure are arranged in one same longitudinal alignment, with the possibility that said sticks could be in different directions but always joined by the gripping device.

Thus, the gripping device functions as a linking or connecting element between two sticks normally of the same length, without ruling out sticks of different lengths.

The characteristic rod structure of the disclosure has the function of being able to be turned over without the user staining their fingers with perfume, in order to take better advantage of the perfume-emitting capacity thereof; wherein a user can grasp the rod structure with their fingers by means of the gripping device.

For this, the gripping device is preferably made of two chosen materials, although they are not exclusive of any other suitable material: a first material is zamak and a second material is wood; wherein both materials are perfume-resistant, since perfumes are very corrosive, which is why these two materials have been initially chosen, without ruling out others.

It has been envisaged that the length at which the gripping device is located with respect to the end of the rod structure that is inside the diffuser container is always greater than the maximum depth of said diffuser container holding the fragrance, such that it will be ensured that the gripping device is always visible (above the mouthpiece thereof) and accessible so that the user can have easy access and therefore be able to remove said rod structure comfortably.

To help better understand this specification, a series of figures is attached as an integral part thereof, in which the object of the disclosure is depicted in an illustrative and non-limiting manner.

### Brief description of the figures

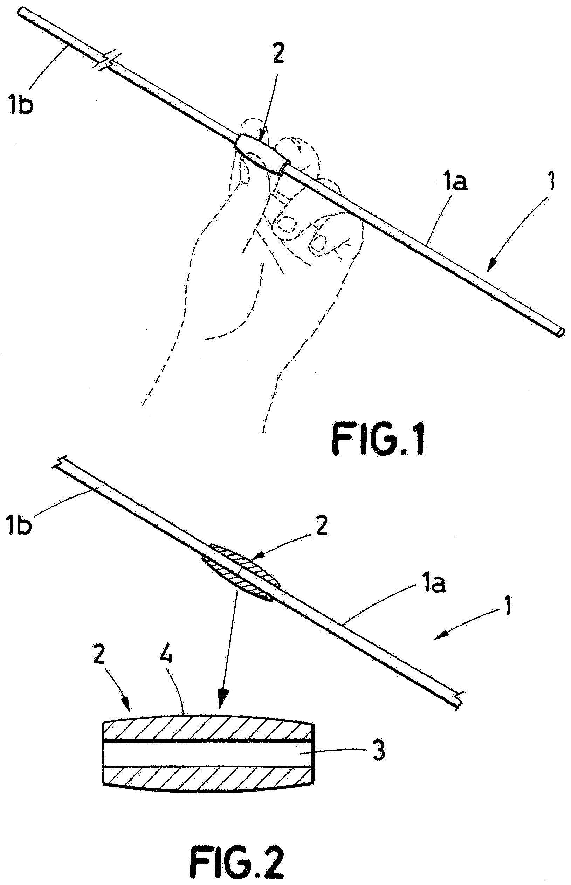

**Figure 1** shows a perspective view of the essence diffusing rod structure with a gripping device, object of the disclosure
**Figure 2** shows an exploded view of the rod structure of the disclosure
**Figure 3** represents a perspective view of the application of the disclosure showing a diffuser container (bottle) with several rod structures housed within said diffuser container and protruding above the mouthpiece thereof.

### Description of an exemplary embodiment of the invention

Considering the numbering adopted in the figures, the essence diffusing rod structure 1 with a gripping device 2 comprises two sticks 1a, 1b that are joined together in e same alignment by the gripping device 2; wherein adjacent end sections of the two rods 1a, 1b are embedded in the gripping device 2.

In the embodiment of the disclosure shown in the figures, the gripping device 2 comprises a tubular body of revolution that includes a cylindrical inner recess 3 and an outer surface 4 with a convex curved generatrix; wherein adjacent end sections of the two rods 1a, 1b of the rod structure 1 fit tightly within said cylindrical inner recess 3.

The two rods 1a, 1b normally have the same length, although it is also possible that they have different lengths.

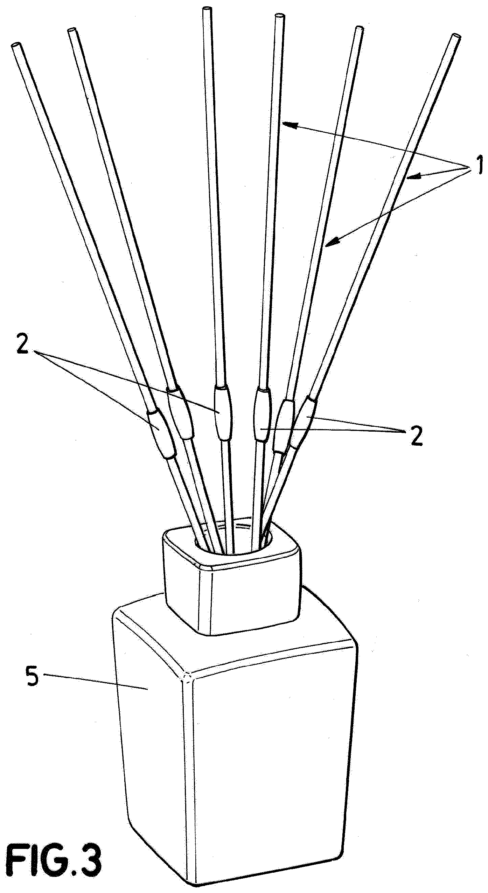

Figure 3 shows a diffuser container 5 (bottle) with several rod structures 1 housed within said diffuser container 5 and protruding above the mouthpiece 5a thereof, such that the gripping device 2 is also above said mouthpiece 5a of the diffuser container 5 so that a user can comfortably hold the corresponding essence diffusing rod structure 1 with their fingers, with the advantage that their skin will not come in contact with the essence that impregnates the rods 1a, 1b.

Obviously, the gripping device 2 is made of a material that does not allow for the capillarity of the essence that impregnates the two rods 1a, 1b.

Another advantage of the disclosure is that during transport, the rod structures 1 can be disassembled into the three elements thereof (pair of rods 1a, 1b and the gripping device 2), which will take up less space and therefore transport will be more cost-effective.

## Claims

1. A set comprising a diffuser container (5) having a mouthpiece (5a) and containing an essence; and an essence diffusing rod structure (1) with a gripping device (2), wherein the rod structure (1) is configured to be inserted through the mouthpiece (5a) of the diffuser container (5), **characterised in that** the essence diffusing rod structure (1) comprises two sticks (1a, 1b) that are joined together by the gripping device (2); wherein adjacent end sections of the two sticks (1a, 1b) are coupled to the gripping device (2); wherein adjacent end sections of the two sticks (1a, 1b) of the rod structure (1) include end portions that are in contact with each other; wherein the gripping device (2) is made of a material that prevents capillarity of the essence that impregnates the two sticks (1a, 1b).

2. The set according to claim 1, wherein the gripping device (2) comprises a tubular body of revolution that includes a cylindrical inner recess (3); wherein the adjacent end sections of the two sticks (1a, 1b) of the rod structure (1) fit tightly within said cylindrical inner recess (3).

3. The set according to claim 2, wherein the tubular body of revolution includes an outer surface (4) with a convex curved generatrix.

4. The set according to any one of the preceding claims, wherein the two sticks (1a, 1b) are arranged in one same longitudinal alignment.

## Patentansprüche

1. Set, umfassend einen Diffusorbehälter (5), der ein Mundstück (5a) aufweist und eine Essenz enthält; und eine Essenzdiffusionsstangenstruktur (1) mit einer Greifvorrichtung (2), wobei die Stangenstruktur (1) dazu ausgelegt ist, durch das Mundstück (5a) des Diffusorbehälters (5) eingeführt zu werden, **dadurch gekennzeichnet, dass** die Essenzdiffusionsstangenstruktur (1) zwei Stäbe (1a, 1b) umfasst, die durch die Greifvorrichtung (2) miteinander verbunden sind; wobei benachbarte Endabschnitte der zwei Stäbe (1a, 1b) mit der Greifvorrichtung (2) gekoppelt sind; wobei benachbarte Endabschnitte der zwei Stäbe (1a, 1b) der Stangenstruktur (1) Endteile umfassen, die miteinander in Kontakt stehen; wobei die Greifvorrichtung (2) aus einem Material hergestellt ist, das die Kapillarität der Essenz verhindert, die die zwei Stäbe (1a, 1b) imprägniert.

2. Set nach Anspruch 1, wobei die Greifvorrichtung (2) einen röhrenförmigen Rotationskörper umfasst, der eine zylindrische innere Aussparung (3) aufweist; wobei die benachbarten Endabschnitte der zwei Stäbe (1a, 1b) der Stangenstruktur (1) eng in die zylindrische innere Aussparung (3) passen.

3. Set nach Anspruch 2, wobei der röhrenförmige Rotationskörper eine Außenfläche (4) mit einer konvex gekrümmten Erzeugenden aufweist.

4. Set nach einem der vorhergehenden Ansprüche, wobei die zwei Stäbe (1a, 1b) in einer gleichen Längsausrichtung angeordnet sind.

## Revendications

1. Ensemble comprenant un récipient de diffuseur (5) ayant une embouchure (5a) et contenant une essence ; et une structure de tige de diffusion d'essence (1) avec un dispositif de préhension (2), dans lequel la structure de tige (1) est configurée pour l'insertion à travers l'embouchure (5a) du récipient de diffuseur (5), **caractérisé en ce que** la structure de tige de diffusion d'essence (1) comprend deux bâtonnets (1a, 1b) qui sont joints ensemble par le dispositif de préhension (2) ; dans lequel des sections d'extrémité adjacentes des deux bâtonnets (1a, 1b) sont couplées au dispositif de préhension (2) ; dans lequel des sections d'extrémité adjacentes des deux bâtonnets (1a, 1b) de la structure de tige (1) incluent des portions d'extrémité qui sont en contact l'une avec l'autre ; dans lequel le dispositif de préhension (2) est fait d'un matériau qui empêche la capillarité de l'essence qui imprègne les deux bâtonnets (1a, 1b).

2. Ensemble selon la revendication 1, dans lequel le dispositif de préhension (2) comprend un corps de révolution tubulaire qui inclut un évidement interne cylindrique (3) ; dans lequel les sections d'extrémité adjacentes des deux bâtonnets (1a, 1b) de la structure de tige (1) s'ajustent étroitement à l'intérieur dudit évidement interne cylindrique (3).

3. Ensemble selon la revendication 2, dans lequel le corps de révolution tubulaire inclut une surface externe (4) avec une génératrice incurvée convexe.

4. Ensemble selon l'une quelconque des revendications précédentes, dans lequel les deux bâtonnets (1a, 1b) sont agencés dans un même alignement longitudinal.
